(19) Europäisches Patentamt / European Patent Office / Office européen des brevets

(11) **EP 4 497 835 B1**

(12) **EUROPEAN PATENT SPECIFICATION**

(45) Date of publication and mention
of the grant of the patent:
**17.06.2026 Bulletin 2026/25**

(21) Application number: **23787625.5**

(22) Date of filing: **10.04.2023**

(51) International Patent Classification (IPC):
*C12Q 1/6883* (2018.01)    *G01N 33/573* (2006.01)
*C12N 15/11* (2006.01)

(52) Cooperative Patent Classification (CPC):
**G01N 33/573; C12Q 1/6883; G01N 33/68;
G01N 33/6893;** C12Q 2600/158; G01N 2333/96494;
G01N 2800/347

(86) International application number:
**PCT/CN2023/087238**

(87) International publication number:
**WO 2023/197981 (19.10.2023 Gazette 2023/42)**

(54) **APPLICATION OF MMP19 IN EARLY DIAGNOSIS OF KIDNEY INJURY, PRODUCT THEREOF, AND METHOD THEREFOR**

VERWENDUNG VON MMP19 BEI DER FRÜHDIAGNOSE VON NIERENLÄSIONEN, PRODUKT DARAUS UND VERFAHREN DAFÜR

APPLICATION DE MMP19 DANS LE DIAGNOSTIC PRÉCOCE D'UNE LÉSION RÉNALE, PRODUIT ASSOCIÉ ET MÉTHODE ASSOCIÉE

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**

(30) Priority: **15.04.2022 CN 202210414753**

(43) Date of publication of application:
**29.01.2025 Bulletin 2025/05**

(73) Proprietor: **Renal Medicine and Biotechnology
Co., Ltd.
Suzhou, Jiangsu 215126 (CN)**

(72) Inventors:
• **ZHENG, Feng**
  **Suzhou, Jiangsu 215126 (CN)**
• **LI, Xuejuan**
  **Suzhou, Jiangsu 215126 (CN)**

(74) Representative: **Bryers Intellectual Property Ltd
Bristol & Bath Science Park
Dirac Crescent, Emerson's Green
Bristol BS16 7FR (GB)**

(56) References cited:
WO-A1-2020/091367     AU-A1- 2016 208 381
CN-A- 103 384 832     CN-A- 110 468 200
CN-A- 112 649 608     US-A1- 2004 101 924

• JAIN SANJAY ET AL: "Expression profiles of congenital renal dysplasia reveal new insights into renal development and disease", PEDIATRIC NEPHROLOGY, SPRINGER VERLAG, BERLIN, DE, vol. 22, no. 7, 1 July 2007 (2007-07-01), pages 962 - 974, XP036958633, ISSN: 0931-041X, [retrieved on 20070701], DOI: 10.1007/S00467-007-0466-6
• KUNUGI SHINOBU ET AL: "Inhibition of matrix metalloproteinases reduces ischemia-reperfusion acute kidney injury", vol. 91, no. 2, 1 February 2011 (2011-02-01), The United States and Canadian Academy of Pathology, Inc., pages 170 - 180, XP093272748, ISSN: 0023-6837, Retrieved from the Internet <URL:https://dul.usage.elsevier.com/doi/> DOI: 10.1038/labinvest.2010.174

EP 4 497 835 B1

- **JING LIU, SANJEEV KUMAR, EGOR DOLZHENKO, GREGORY F. ALVARADO, JINJIN GUO, CAN LU, YIBU CHEN, MENG LI, MARK C. DESSING, RIANA K. PA: "Molecular characterization of the transition from acute to chronic kidney injury following ischemia/ reperfusion", JCI INSIGHT, vol. 2, no. 18, 21 September 2017 (2017-09-21), XP055527516, DOI: 10.1172/jci.insight.94716**

**Description**

**FIELD OF TECHNOLOGY**

The present invention belongs to the technical field of biological diagnosis and particularly relates to use of MMP19 as a biomarker related to early kidney injury in the early diagnosis of kidney injury.

**BACKGROUND**

There are currently about five million patients with uremia in China, and about 400,000 new cases are added each year. If we conservatively calculate that each patient needs an investment of about 100,000 yuan per year, the current five million patients will require an annual investment of about 500 billion yuan, most of which is the expense for dialysis machines, consumables and medicines imported from abroad.

Early identification and intervention to slow down the development of chronic kidney disease and uremia in people at high risk is the key to solving this major health and economic problem. Early kidney injury includes functional and structural damage to the glomerular filtration membrane and renal tubules, caused by various pathogenic pathways such as various inflammations, metabolic abnormalities and immune injury. People at high risk of chronic kidney disease, such as diabetes, hypertension, and a family history of kidney disease, experience slow and unnoticeable progressive deterioration of kidney lesions.

The detection of early kidney injury indicators plays an important role in the early diagnosis and early intervention of kidney disease. Classic biomarkers such as creatinine and/or urea nitrogen are indicators for monitoring kidney function, but cannot be used as indicators for detecting early kidney injury. Especially in the acute stage, kidney injury is not correlated with changes in these classic indicators.

Therefore, it is urgent to find a flexible, convenient, non-invasive, highly sensitive and specific method for the early detection of kidney injury, so as to achieve early diagnosis of kidney injury.

AU2016208381A1 provides a method of diagnosing the existence of a kidney disorder in a feline comprising measuring the level of expression of one or more hiomarkers selected from the group consisting of lunican; collagen alpha 1(Ul) chain, variant 12; decorin; secreted frizzled-related protein 2; retinol binding protein 5; MMP-2; MMP-7; and MMP-19, in a biological sample from the feline, wherein differences in expression of the one or nore biornarkers in the sample relative to a control value for expression in a sample from a normal animal indicate the existence of a kidney disorder; a method of treating a feline so diagnosed; and compositions, reagents and kits for carrying out the specified methods.

JAIN SANJAY ET AL: "Expression profiles of congenital renal dysplasia reveal new insights into renal development and disease" discloses that expression of selected gene products(BMP7,renin, and MMP7) was further confirmed in parallel sections and in several normal and human dysplastic kidneys, supporting the role of these genes as putative RD biomarkers.

KUNUGI SHINOBU ET AL: "Inhibition of matrix metalloproteinases reduces ischemia-reperfusion acute kidney injury" discloses the use of MMP-2 as marker for AKI.

SUMMARY OF THE INVENTION

An objective of the present invention is to provide use of a reagent for detecting a biomarker in a sample in the preparation of a product for early diagnosis of acute kidney injury, so as to determine whether a subject has acute kidney injury or is at risk of kidney disease in an early, accurate, rapid and non-invasive manner.

To solve the above technical problems, the present invention adopts the following technical solution:

The present invention provides use of a reagent for detecting a biomarker in a sample in the preparation of a product for early diagnosis of acute kidney injury, wherein the biomarker is MMP19.

Preferably, the reagent for detecting a biomarker in a sample includes a reagent for detecting the expression level and/or activity of the biomarker's mRNA in the sample, and/or, the reagent for detecting a biomarker in a sample includes a reagent for detecting the expression level and/or activity of the biomarker's protein in the sample.

Further preferably, the reagent is one or more of a primer for specifically amplifying the biomarker, a probe for specifically identifying the biomarker, a conjugate that specifically binds to a protein encoded by the biomarker, or a reagent capable of detecting the activity of the biomarker.

Still further preferably, the conjugate includes one or more of an antibody, a functional antibody fragment or an antibody conjugate that specifically binds to a protein encoded by the biomarker.

Preferably, the expression level and/or activity of the biomarker MMP19 in the test sample detected with the reagent for the biomarker in the test sample is compared with that in a normal control, and if the expression level and/or activity of the biomarker MMP19 is increased, the subject is determined to have acute kidney injury or a tendency to suffer from kidney disease or a recurrence of kidney disease or a poor prognosis of kidney disease.

Preferably, the test sample is blood and/or urine.

Further preferably, the test sample is a serum sample.

Preferably, the acute kidney injury is tubulointerstitial injury.

The present invention further provides a method for early diagnosis of acute kidney injury.

Specifically, the method includes the following steps:

1) detecting the expression level and/or activity of a biomarker MMP19 in the test sample from the subject;
2) correlating the detected expression level and/or activity of the biomarker MMP19 with the disease or the risk of disease of the subject; and
3) if the expression level and/or activity of the biomarker MMP19 is increased compared with a normal control, determining that the subject has acute kidney injury or a tendency to kidney disease or a recurrence of kidney disease or a poor prognosis of kidney disease.

The kit used in the present invention may be an existing MMP19 test kit.

The primer for specifically amplifying the biomarker MMP19 and/or the probe for specifically identifying the biomarker MMP19, described in the present invention, may be designed based on MMP19 sequence information and prepared by chemical synthesis, and the conjugate that specifically binds to a protein encoded by the biomarker MMP19 may be an antibody of any structure, size, source, and the like or a fragment of the antibody as long as the antibody or the fragment thereof can bind to matrix metallopeptidase 19.

Compared with the prior art, the present invention has the following advantages:

The present invention discovers for the first time that the MMP19 can be used as a biomarker related to early acute kidney injury, and also provides the application of the reagent for detecting a biomarker in a sample in the preparation of a product for early diagnosis of acute kidney injury, so as to determine whether a subject has acute kidney injury or is at risk of kidney disease in an early, accurate, quick and non-invasive manner, and to perform early intervention of kidney disease.

**BRIEF DESCRIPTION**

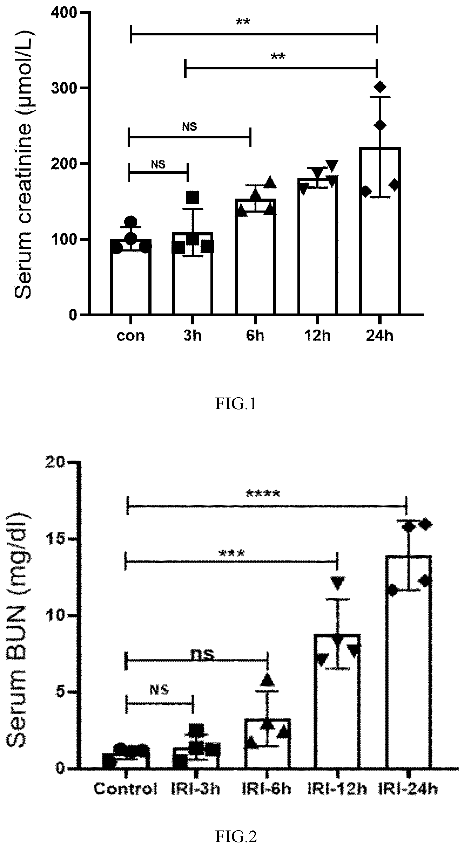

FIG. 1 shows serum creatinine levels of mice at different time points after acute kidney injury (AKI) modeling in Example 1;

FIG. 2 shows blood urea nitrogen levels of mice at different time points after AKI modeling in Example 1;

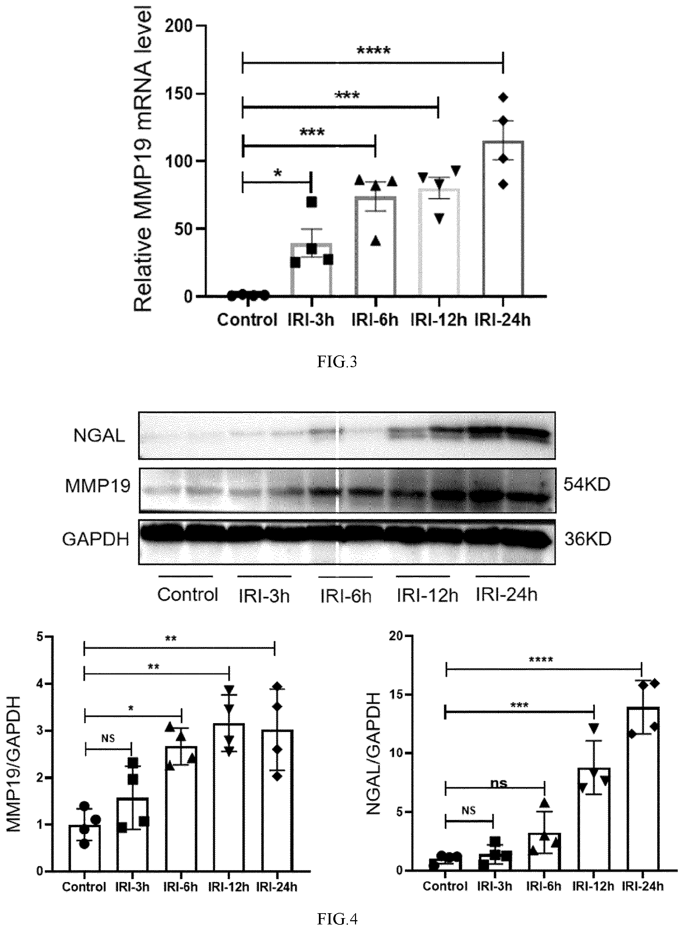

FIG. 3 shows mRNA levels of MMP19 in serum of mice at different time points after AKI modeling in Example 1;

FIG. 4 shows experimental results at different time points after AKI modeling in Example 1, where graphs at the top show results of the protein expression levels of MMP19, AKI marker protein NGAL and GAPDH in serum of mice, detected by Western Blot; the left graph at the bottom shows results of comparison of MMP19 content in serum with MMP19 level after correction for GAPDH content; the right graph at the bottom shows results of comparison of NGAL content in serum with NGAL level after correction for GAPDH content;

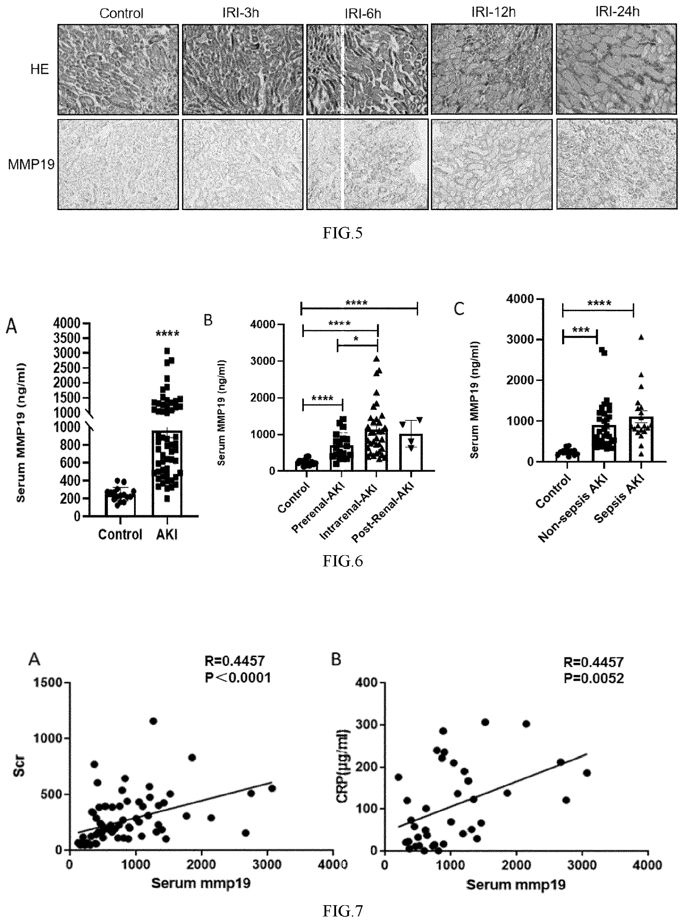

FIG. 5 shows kidney tissue sections and immunohistochemistry results at different time points after AKI modeling in Example 1, where images at the top show (HE) staining results for pathological analysis of kidney, and images at the bottom show immunohistochemistry results of kidney tissue;

FIG. 6 shows test results of clinical AKI samples in Example 2: A) results of protein expression levels of serum MMP19 of healthy control and AKI samples; B) results of protein expression levels of serum MMP19 of AKI samples classified by different causes of disease; C) results of the protein expression levels of serum MMP19 of AKI samples classified by whether Sepsis occurs;

FIG. 7 shows analysis results of correlation between serum MMP19 protein level in clinical AKI samples and clinical indicators in Example 2: A) analysis results of correlation between serum MMP19 protein content and patient's serum creatinine; B) analysis results of correlation between serum MMP19 protein content and patient's C-reactive protein;

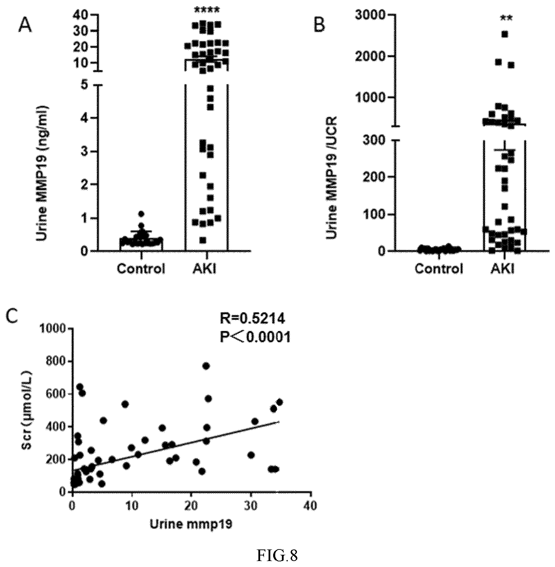

FIG. 8 shows results of protein expression levels of MMP19 in urine samples of AKI patients of different causes: A) results of protein expression levels of urine MMP19 in healthy control and AKI samples; B) comparison results of MMP19 content in urine with MMP19 level after correction for patient's urine creatinine; C) analysis results of correlation between urine MMP19 content and serum creatinine;

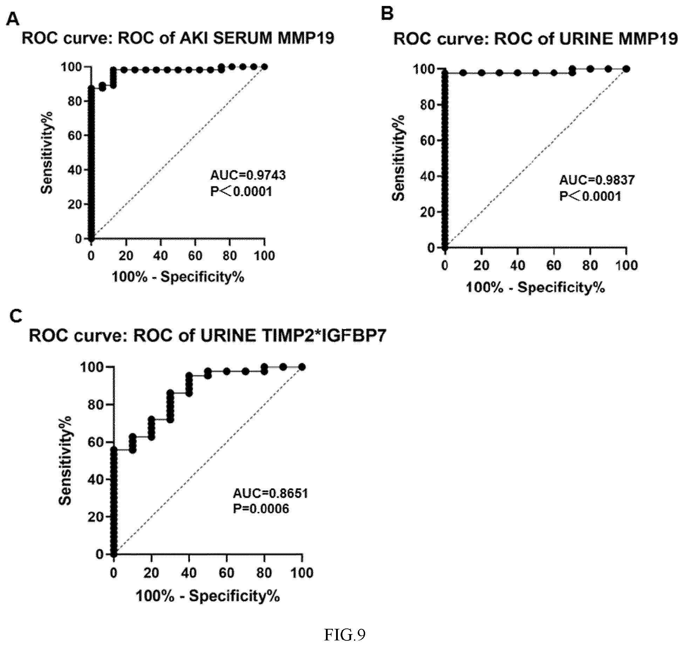

FIG. 9 shows ROC curves of MMP19 and [TIMP2]*[IGFBP7] for the diagnosis of AKI: A) ROC curve of serum MMP19 protein for the diagnosis of AKI; B) ROC curve of urine MMP19 protein for the diagnosis of AKI; C) ROC curve of urine [TIMP2]*[IGFBP7] (FDA-approved AKI biological markers) for the diagnosis of AKI.

## DETAILED DESCRIPTION OF THE INVENTION

The invention is further described below in conjunction with embodiments. However, the invention is not limited to the following embodiments. The implementation conditions used in the embodiments may be further adjusted according to the different requirements of specific use, and the implementation conditions not specified are conventional conditions in this industry. The technical features involved in various embodiments of the invention may be combined with each other as long as they do not conflict with each other.

Since creatinine and/or urea nitrogen cannot be used as indicators for detecting early kidney injury and in the acute stage, kidney injury is not correlated with changes in creatinine and/or urea nitrogen indicators. Therefore, the inventors conducted a lot of research and experimental verification and screened out the biomarker MMP19 suitable for early kidney injury.

In the present invention, the "biomarker" refers to a compound, preferably a gene. The concentration and/or content of the biomarker in a biological sample from a subject with a first phenotype (e.g., the presence of kidney injury) is different (i.e., increased or decreased) from that in a biological sample from a subject with a second phenotype (e.g., no kidney injury). The concentration and/or content refers to the protein/gene expression concentration or protein/gene content.

In the present invention, the concentration and/or content of the biomarker in the above two groups may differ at any level. However, the difference generally exists at the following levels, such as an increase of at least 5%, at least 10%, at least 15%, at least 20%, at least 25%, at least 30%, at least 35%, at least 40%, at least 45%, at least 50%, at least 55%, at least 60%, at least 65%, at least 70%, at least 75%, at least 80%, at least 85%, at least 90%, at least 95%, at least 100%, at least 110%, at least 120%, at least 130%, at least 140%, at least 150%, or more. Preferably, the difference is statistically significant ($P<0.05$).

In the present invention, the "test sample" includes, but is not limited to, serum, plasma, whole blood, blood-derived cells, urine, sweat, kidney tissue and combinations thereof. As a preferred embodiment, the sample is selected from serum, urine or kidney tissue.

In the present invention, the "subject" refers to any animal, including human and non-human animals. Non-human animals include all vertebrates, such as, mammals, such as non-human primates (especially higher primates), sheep, dogs, rodents (such as mice or rats), guinea pigs, goats, pigs, cats, rabbits, cattle, and any livestock or pets; and non-mammals, such as chickens, amphibians, reptiles, etc. In a preferred embodiment, the subject is a human.

In the present invention, the step of correlating the expression level of the biomarker with a certain possibility or risk can be implemented in different ways. Preferably, the detected protein concentrations are mathematically combined and related to the underlying diagnosis question.

Preferably, the logarithmic function used to correlate the biomarker level to the disease preferably employs an algorithm developed and obtained by applying a statistical method. For example, suitable statistical methods are discriminant analysis (DA) (i.e., linear, quadratic, and regular DA), kernel method (i.e., SVM), nonparametric methods (i.e., k-nearest neighbor classifier), PLS (partial least squares), tree-based methods (i.e., logistic regression, CART, random forest method, and boosting/bagging methods), generalized linear models (i.e., logistic regression), principal component-based methods (i.e., SIMCA), generalized additive models, fuzzy logic-based methods, neural network and genetic algorithm-based methods, and correlation analysis.

The area under the curve (AUC) of receiver operator characteristic is an indicator of the performance or accuracy of a diagnosis procedure. The accuracy of a diagnosis method is described by its receiver operator characteristic (ROC). The ROC plot is a line plot of all sensitivity/specificity pairs resulting from continuously varying the decision threshold over the entire range of data observed.

The clinical performance of a laboratory test item depends on its diagnosis accuracy and the diagnosis accuracy refers to the ability of a test item to distinguish a state of the subject in two or more clinical states.

In the specific embodiments of the present invention, the raw materials used can be obtained commercially.

In a specific embodiment of the present invention, the NCBI accession numbers of MMP19 are NM 002429.6 and NM 001272101.2,

the detection of human MMP19 uses an ELISA kit from Biorbyt in the UK: Cat No. orb551482;
the detection of mouse MMP19 uses an ELISA kit from Biorbyt in the UK: Cat No. orb777030;
the MMP19 antibody is purchased from novus: Cat No: NBP2-17311;
the NCBI accession numbers of GAPDH are NM 001289726.1 and NM 008084.3;
the detection of GAPDH uses proteintech GAPDH Monoclonal antibody, Cat No. 60004-1-Ig;
the α-SMA antibody is purchased from Cell Signaling Technology, Cat No. 19245S;
the NCBI accession number of TIMP2 is NM 003255.5; the detection of TIMP2 uses an ELISA kit from Wuhan CUSABIO, Cat No. CSB-E04733h;
the NCBI accession numbers of NGAL are NM 001360406.1 and NM 021551.4, and the antibody used for detection is ABCAM, Cat No. ab216462;

the NCBI accession numbers of IGFBP7 are NM 001553.3 and NM 001253835.2, and the detection of IGFBP7 uses an ELISA kit from Wuhan CUSABIO, Cat No. CSB-E17249h;

serum creatinine and urine creatinine were tested using a creatinine test kit purchased from Nanjing Jiancheng Bioengineering Research Institute, by a sarcosine oxidase method, Cat No. C011-2-1;

blood urea nitrogen (BUN) was tested using test kits purchased from Nanjing Jiancheng Bioengineering Research Institute, using a urease method, Cat No. C013-2-1;

C-reactive protein was tested by immunochromatography;

the eGFR was tested by a simplified MDRD equation modified by the Chinese:

$$eGFR\ (ml/(min*1.73m^2)) = 186X\ (Scr)^{-1.154}X\ (age)^{-0.203}X\ (0.742\ female)$$

Note: eGFR refers to glomerular filtration rate, ml/(min*1.73m$^2$); Scr is serum creatinine (mg/dl); age is in years; weight is in kg.

Example 1:

Acute Kidney Injury (AKI) mouse experiment

Renal ischemia reperfusion injury (IRI) model in mice

Healthy male wild-type C57BL/6 mice of the same batch, about 8 weeks old, were selected and randomly divided into five groups, with 4 mice in each group. The five groups were a control group (Control or con), a group of mice with the disease model for 3 hours (3h or IRI-3h), a group of mice with the disease model for 6 hours (6h or IRI-6h) , a group of mice with the disease model for 12 hours (12h or IRI-12h), and a group of mice with the disease model for 24 hours (24h or IRI-24h), respectively. All surgical instruments were sterilized by high pressure, and the abdomen of the mice was depilated in advance.

Preparation of anesthetics: 25 g of tribromoethanol and 15.5 mL of tert-amyl alcohol were mixed to obtain a 1600 mg/mL concentrated tribromoethanol stock solution. After complete dissolution, the stock solution was stored in the dark. The stock solution needs to be diluted into a 20 mg/mL working solution (with saline) before use.

Anesthesia: 20 mg/mL tribromoethanol working solution was injected intraperitoneally at a dose of 350 mg/kg (17.5 $\mu$l/g mouse body weight) to anesthetize the mice. The toes of the mice were gently pinched with tweezers to determine whether the mice entered a deep anesthesia state.

Surgery for the model groups: After entering a deep anesthesia, the mice were fixed in a supine position on a 37°C constant temperature sterile operating table, the abdominal skin was disinfected, and the abdomen was opened. The bilateral kidneys were located with the help of cotton swabs, the fat tissue around the renal pedicles was separated, and after being freed, the renal pedicles were then clamped with vascular clamps for 40 minutes. After the vascular clamps were removed, it was observed with the naked eye that the color of the kidneys returned from purple-black to their original color. After successful reperfusion in kidneys, the incision was sutured and disinfected with iodine, and 500ul of normal saline was injected intraperitoneally for fluid replacement after the surgery. After waking up, the mice were returned to the cage and given free access to food and water.

Surgery for the control group: During the ischemia reperfusion surgery on the first day, the surgery operations were the same as those for the surgery groups except that the blood vessels were not clamped.

Urine samples were collected, using a mouse metabolic cage, from the mice in the model groups at 3 hours (IRI-3h), 6 hours (IRI-6h), 12 hours (IRI-12h), and 24 hours (IRI-24h) after modeling, and from the mice in the control group at 24 hours after sham operation and centrifuged at a low temperature (4°C, 3000 rpm, 10 min). After the urine collection, the mouse was killed. The mouse was anesthetized successfully and fixed on a killing board. The abdominal cavity was opened, and blood was collected from the inferior vena cava of the mouse with a 1 ml syringe. The blood collection speed was adjusted according to the filling condition of the inferior vena cava. The needle of the syringe was carefully pulled out, and the blood was injected into an EP tube along the wall of the tube. After standing at room temperature for 1 hour, the blood was centrifuged at 3000 rpm for 10 min, and the supernatant was aspirated and subpackaged. The chest cavity of the mouse was opened, the heart was exposed, the right auricle was cut, and an intravenous infusion needle connected to a 20 ml syringe was inserted from the left ventricle of the mouse. Sterile PBS was perfused until the kidney turned white, the renal capsule was then peeled off, the kidney was removed, and kidney tissue about 3 mm thick in the middle was cut along the short axis of the kidney with a blade, and placed in an EP tube (containing 4% paraformaldehyde) for fixation for more than 24 h, and stored at 4 °C in a refrigerator. Then, kidney tissue about 3 mm thick was cut and then placed in an EP tube filled with an optimal cutting temperature compound (OCT) and the EP tube was then placed in liquid nitrogen. The remaining kidney tissues were subpackaged and placed in liquid nitrogen for subsequent extraction of RNA and tissue protein. The retained urine, serum, kidney tissue protein, and RNA samples of the mouse were all stored at -80 °C in a refrigerator.

At different time points after AKI modeling, the levels of serum creatinine of mice are shown in FIG. 1, and the levels of serum blood urea nitrogen of mice are shown in FIG. 2. Compared with the control group, the model groups had increased serum creatinine and urea nitrogen in mice 12 hours after ischemia reperfusion.

At different time points after AKI modeling, the mRNA levels of MMP19 in serum of mice are shown in FIG. 3. The gene expression of MMP19 in kidney tissue was up-regulated at different time points after AKI modeling. The degree of upregulation increased with time. The mRNA level of MMP19 increased 3 hours after surgery, earlier than the increase in serum creatinine and urea nitrogen of mice.

At different time points after AKI modeling, Western Blot was performed to detect the protein expression levels of MMP19 gene, NGAL gene and GAPDH gene in mouse serum (FIG. 4). The protein expression level of MMP19 gene began to up-regulate 3 hours after modeling, which was significantly earlier than the protein expression level of NGAL gene.

The kidney tissue of mice was subjected to (HE) staining for pathological analysis, and the results are shown in the images at the top of FIG. 5. Immunohistochemistry was performed to detect changes in the protein expression distribution of MMP19, and the results are shown in the images at the bottom of FIG. 5. HE staining showed that histopathological damage in the kidney began to occur 6 hours after modeling, while the immunohistochemistry results of MMP19 showed that the expression of MMP19 was significantly up-regulated 3 hours after modeling, earlier than the pathological damage in the kidney.

**Example 2:**

Clinical AKI sample detection experiment

Collection of serum and urine samples from AKI patients: Morning urine/random urine was collected. The urine sample was centrifuged at a low temperature (4 °C, 3000 rpm, 10 min). The urine supernatant was taken and subpackaged at 1 mL/tube and stored at -80 °C in a refrigerator. In addition, a tube of serum sample from each patient undergoing renal puncture biopsy was collected on the day of biopsy, where the vacuum blood collection tube with biochemical serum coagulant was used for specimen collection. Immediately after blood collection, the sample was mixed homogeneously by overturning the tube up and down for 8 times, and then allowed to stand for 15-20 min, and then centrifuged at a low temperature (4 °C, 3000 rpm, 10 min). The supernatant was then taken and subpackaged at 20 $\mu$l/tube, and then stored at -80 °C in a refrigerator.

Samples were collected from patients with clinical diagnosis of acute kidney injury. The patients selected should meet the following conditions: 1. Within 48 hours, the average of serum creatinine indicator is greater than 26.4 $\mu$mol/L. 2. Within one week, the serum creatinine level increases to more than 1.5 times the baseline level (the basic value of the patient himself). 3. Urine volume is less than 0.5 ml/h for 3-4 consecutive days. Patients will be selected in the group as long as their test results meet any one of these conditions. Blood and urine samples were collected from the patients in the group.

Collection of samples from the healthy control group: Samples were collected from patients undergoing physical examinations at the physical examination center. The patients selected should meet the following conditions: their age and sex should match those of the patients undergoing renal puncture biopsy and patients with acute kidney injury, and they should have normal renal function test results. The samples, collected in the laboratory after testing, and the steps of sample collection, processing and storage were the same as above.

In this example, we collected 16 serum samples from healthy patients having physical examination as the healthy control group and 56 serum samples from AKI samples.

The serum MMP19 levels of the healthy control group and acute kidney injury samples are shown in FIG. 6A. Compared with the healthy control group, the serum MMP19 levels in patients in the AKI disease group were significantly up-regulated. The average of serum MMP19 level in the control group was 248.9$\pm$18.91 ng/ml, and the average of serum MMP19 level in AKI patients was 966.4$\pm$83.14 ng/ml.

Patients in the AKI disease group were classified according to different causes of the disease. The serum MMP19 levels of AKI samples classified by different causes of the disease are shown in FIG. 6B. There were 19 patients with Prerenal-AKI, with an average serum MMP19 level of 695$\pm$81.49 ng/ml; 34 patients with Intrarenal-AKI, with an average serum MMP19 level of 1130$\pm$120.8 ng/ml; and 4 patients with Post-Renal-AKI, with an average serum MMP19 level of 1023$\pm$183.4 ng/ml. One of the cases has both Prerenal-AKI and Intrarenal-AKI.

The AKI patients were divided into Sepsis AKI and Non-sepsis AKI according to whether they had Sepsis at the same time. The serum MMP19 levels are shown in FIG. 6C. There were 36 cases of Non-sepsis AKI with an average serum MMP19 level of 913.6$\pm$95.34 ng/mL and 20 cases of Sepsis AKI with an average serum MMP19 level of 1091$\pm$186.2 ng/mL.

The prism 8.0 correlation was used to analyze the correlation between the serum MMP19 content and the patient's serum creatinine, urea nitrogen, CRP, etc. The results are shown in FIG. 7. The serum MMP19 content was positively correlated with the patient's serum creatinine (FIG. 7A), and the serum MMP19 content was positively correlated with the patient's C-reactive protein (CRP) (FIG. 7B). The content of MMP19 in serum also showed a correlation with blood urea nitrogen, but there was no statistical difference.

In addition, urine samples were collected from 43 AKI patients of different causes and from 25 age- and sex-matched controls and then tested for the content of MMP19. The results are shown in FIG. 8.

Referring to FIG. 8A, compared with the healthy control group, the secretion of MMP19 in the urine of AKI patients in the AKI disease group increased significantly; specifically, the average of urine MMP19 content in the control group was $0.3896 \pm 0.04234$ ng/ml; the average of urine MMP19 content of AKI patients was $12.44 \pm 1.708$ ng/ml. FIG. 8B shows adjusted MMP19 levels based on the patients' urine creatinine levels , where the average of MMP19 level in the healthy control group was $4.648 \pm 0.6982$, while the average of urine MMP19 content of AKI patients was $346.8 \pm 80.91$. The prism 8.0 correlation was used to analyze the correlation between the urine MMP19 content and the patient's clinical indicators (FIG. 8C). The urine MMP19 content was positively correlated with the patient's serum creatinine.

The area under the ROC curve (AUC) was used to evaluate the sensitivity and specificity of MMP19 as a diagnostic marker and compared with urine [TIMP2]*[IGFBP7] (FDA-approved AKI biomarkers). The results are shown in FIG. 9. The results show that the area under the ROC curve for serum MMP19 in the diagnosis of AKI is 0.9743 (FIG. 9A), and the area under the ROC curve for urine MMP19 in the diagnosis of AKI is 0.9837 (FIG. 9B), while the area under the ROC curve for the FDA-approved AKI biomarkers urine [TIMP2]*[IGFBP7] (TIMP2 content multiplied by IGFBP7 content) in the diagnosis of AKI is 0.8651 (FIG. 9C, using the collected urine samples from 43 AKI patients and 10 age- and sex-matched control samples). Urine MMP19 showed a higher efficacy in the diagnosis of AKI. Therefore, MMP19 is suitable as an effective biomarker for the early diagnosis of acute kidney injury.

In summary, MMP19 can be used as a biomarker for early diagnosis of acute kidney injury. An existing MMP19 test kit or a self-developed MMP19 test kit was used as an early diagnosis kit for kidney failure.

## Claims

1. Use of a reagent for detecting a biomarker in a sample in the preparation of a product for early diagnosis of acute kidney injury, **characterized in that** the biomarker is MMP19.

2. The use according to claim 1, **characterized in that** the reagent for detecting a biomarker in a sample comprises a reagent for detecting the expression level and/or activity of the biomarker's mRNA in the sample, and/or, the reagent for detecting a biomarker in a sample comprises a reagent for detecting the expression level and/or activity of the biomarker's protein in the sample.

3. The use according to claim 2, **characterized in that** the reagent is one or more of a primer for specifically amplifying the biomarker, a probe for specifically identifying the biomarker, a conjugate that specifically binds to a protein encoded by the biomarker, or a reagent capable of detecting the activity of the biomarker.

4. The use according to claim 3, **characterized in that** the conjugate comprises one or more of an antibody, a functional antibody fragment or an antibody conjugate that specifically binds to the protein encoded by the biomarker.

5. The use according to claim 1, **characterized in that**, the expression level and/or activity of the biomarker MMP19 in a test sample from a subject is compared with that in a normal control, and if the expression level and/or activity of the biomarker MMP19 is increased, the subject is determined to have acute kidney injury or a tendency to suffer from kidney disease or a recurrence of kidney disease or a poor prognosis of kidney disease.

6. The use according to claim 1, **characterized in that** a test sample is blood and/or urine.

7. The use according to claim 1, **characterized in that** the acute kidney injury is tubulointerstitial injury.

8. A method for early diagnosis of acute kidney injury, **characterized in that**, it comprises: detecting the expression level and/or activity of a biomarker MMP19 in a test sample from a subject; and comparing a detection result with a normal control, and if the expression level and/or activity of the biomarker MMP19 in the test sample is increased, determining that the subject has acute kidney injury or a tendency to kidney disease or a recurrence of kidney disease or a poor prognosis of kidney disease.

## EP 4 497 835 B1

**Patentansprüche**

1. Verwendung eines Reagenzes zum Nachweis eines Biomarkers in einer Probe bei der Herstellung eines Produkts zur Frühdiagnose einer akuten Nierenschädigung, **dadurch gekennzeichnet, dass** der Biomarker MMP19 ist.

2. Verwendung nach Anspruch 1, **dadurch gekennzeichnet, dass** das Reagenz zum Nachweis eines Biomarkers in einer Probe ein Reagenz zum Nachweis des Expressionsniveaus und/oder der Aktivität der mRNA des Biomarkers in der Probe umfasst, und/oder dass das Reagenz zum Nachweis eines Biomarkers in einer Probe ein Reagenz zum Nachweis des Expressionsniveaus und/oder der Aktivität des Proteins des Biomarkers in der Probe umfasst.

3. Verwendung nach Anspruch 2, **dadurch gekennzeichnet, dass** das Reagenz eines oder mehrere von Folgendem ist: ein Primer zur spezifischen Amplifikation des Biomarkers, eine Sonde zur spezifischen Identifizierung des Biomarkers, ein Konjugat, das spezifisch an ein vom Biomarker kodiertes Protein bindet, oder ein Reagenz, das in der Lage ist, die Aktivität des Biomarkers nachzuweisen.

4. Verwendung nach Anspruch 3, **dadurch gekennzeichnet, dass** das Konjugat eines oder mehrere von Folgendem umfasst: einen Antikörper, ein funktionelles Antikörperfragment oder ein Antikörperkonjugat, das spezifisch an das vom Biomarker kodierte Protein bindet.

5. Verwendung nach Anspruch 1, **dadurch gekennzeichnet, dass** das Expressionsniveau und/oder die Aktivität des Biomarkers MMP19 in einer Testprobe von einem Subjekt mit demjenigen in einer Normalkontrolle verglichen wird, und wenn das Expressionsniveau und/oder die Aktivität des Biomarkers MMP19 erhöht ist, festgestellt wird, dass das Subjekt eine akute Nierenschädigung oder eine Neigung, an einer Nierenerkrankung zu leiden, oder ein Rezidiv einer Nierenerkrankung oder eine schlechte Prognose einer Nierenerkrankung aufweist.

6. Verwendung nach Anspruch 1, **dadurch gekennzeichnet, dass** eine Testprobe Blut und/oder Urin ist.

7. Verwendung nach Anspruch 1, **dadurch gekennzeichnet, dass** die akute Nierenschädigung eine tubulointerstitielle Schädigung ist.

8. Verfahren zur Frühdiagnose einer akuten Nierenschädigung, **dadurch gekennzeichnet, dass** es Folgendes umfasst: Nachweisen des Expressionsniveaus und/oder der Aktivität eines Biomarkers MMP19 in einer Testprobe von einem Subjekt; und Vergleichen eines Nachweisergebnisses mit einer Normalkontrolle, und wenn das Expressionsniveau und/oder die Aktivität des Biomarkers MMP19 in der Testprobe erhöht ist, Feststellen, dass das Subjekt eine akute Nierenschädigung oder eine Neigung zu einer Nierenerkrankung oder ein Rezidiv einer Nierenerkrankung oder eine schlechte Prognose einer Nierenerkrankung aufweist.

**Revendications**

1. Utilisation d'un réactif pour détecter un biomarqueur dans un échantillon dans la préparation d'un produit pour le diagnostic précoce d'une lésion rénale aiguë, **caractérisée en ce que** le biomarqueur est MMP19.

2. Utilisation selon la revendication 1, **caractérisée en ce que** le réactif pour détecter un biomarqueur dans un échantillon comprend un réactif pour détecter le niveau d'expression et/ou l'activité de l'ARNm du biomarqueur dans l'échantillon, et/ou, le réactif pour détecter un biomarqueur dans un échantillon comprend un réactif pour détecter le niveau d'expression et/ou l'activité de la protéine du biomarqueur dans l'échantillon.

3. Utilisation selon la revendication 2, **caractérisée en ce que** le réactif est un ou plusieurs parmi une amorce pour amplifier spécifiquement le biomarqueur, une sonde pour identifier spécifiquement le biomarqueur, un conjugué qui se lie spécifiquement à une protéine codée par le biomarqueur, ou un réactif capable de détecter l'activité du biomarqueur.

4. Utilisation selon la revendication 3, **caractérisée en ce que** le conjugué comprend un ou plusieurs parmi un anticorps, un fragment d'anticorps fonctionnel ou un conjugué d'anticorps qui se lie spécifiquement à la protéine codée par le biomarqueur.

5. Utilisation selon la revendication 1, **caractérisée en ce que** le niveau d'expression et/ou l'activité du biomarqueur MMP19 dans un échantillon à tester provenant d'un sujet est comparé à celui d'un témoin normal, et si le niveau d'expression et/ou l'activité du biomarqueur MMP19 est augmenté(e), il est déterminé que le sujet présente une lésion rénale aiguë ou une tendance à souffrir d'une maladie rénale ou d'une récidive de maladie rénale ou d'un mauvais pronostic de maladie rénale.

**6.** Utilisation selon la revendication 1, **caractérisée en ce qu'**un échantillon à tester est du sang et/ou de l'urine.

**7.** Utilisation selon la revendication 1, **caractérisée en ce que** la lésion rénale aiguë est une lésion tubulo-interstitielle.

**8.** Procédé pour le diagnostic précoce d'une lésion rénale aiguë, **caractérisé en ce qu'**il comprend : la détection du niveau d'expression et/ou de l'activité d'un biomarqueur MMP19 dans un échantillon à tester provenant d'un sujet ; et la comparaison d'un résultat de détection avec un témoin normal, et si le niveau d'expression et/ou l'activité du biomarqueur MMP19 dans l'échantillon à tester est augmenté(e), la détermination que le sujet présente une lésion rénale aiguë ou une tendance à une maladie rénale ou à une récidive de maladie rénale ou à un mauvais pronostic de maladie rénale.

FIG.1

FIG.2

FIG.3

FIG.4

FIG.5

FIG.6

FIG.7

FIG.8

FIG.9

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- AU 2016208381 A1

**Non-patent literature cited in the description**

- **JAIN SANJAY et al.** *Expression profiles of congenital renal dysplasia reveal new insights into renal development and disease*

- **KUNUGI SHINOBU et al.** *Inhibition of matrix metalloproteinases reduces ischemia-reperfusion acute kidney injury*